# EUROPEAN PATENT APPLICATION

(11) **EP 4 390 899 A1**
(43) Date of publication of application: **26.06.2024**
(21) Application number: 21954165.3
(22) Date of filing: 18.08.2021
(51) Int. Cl.: G09B 23/34, C12N 5/071

(54) **ENDOMETRIUM MODEL, METHOD FOR PRODUCING ENDOMETRIUM MODEL, AND ENDOMETRIAL IMPLANTATION MODEL**

(71) Applicant: Tohoku University, Sendai-shi, Miyagi 980-8577 (JP)
(72) Inventor: ARIMA, Takahiro, Sendai-shi, Miyagi 980-8577 (JP); SHIBATA, Shun, Sendai-shi, Miyagi 980-8577 (JP); OKAE, Hiroaki, Sendai-shi, Miyagi 980-8577 (JP)
(74) Representative: Cabinet Becker et Associés
(86) International application number: PCT/JP2021/030100
(87) International publication number: WO 2023/021591

(57) **Abstract**

There is provided an endometrial model including: a stromal extracellular matrix-containing gel; and endometrial epithelial cells, wherein at least some of the endometrial epithelial cells are exposed on a surface of the gel, and wherein the at least some of the endometrial epithelial cells exposed on the surface of the gel continuously form a luminal structure and a non-luminal structure. Also, a method for preparing the endometrial model is also provided. Further, an endometrial implantation model including: the endometrial model and an embryonic cell is provided.

## Description

### Technical Field

The present invention relates to an endometrial model, a method for preparing an endometrial model, and an endometrial implantation model.

### Background Art

The endometrium is an epithelial tissue present in the uterus and is a tissue necessary for the establishment of pregnancy. During reproductive periods of human adults, functional layers of the endometrium repeat monthly cycles of regeneration, differentiation, and desquamation. After menstruation, the functional layers of the endometrium proliferate in an estrogen-dependent manner, followed by mucosal regeneration, and differentiation in a progesterone-dominant secretory phase. About 7 days after ovulation, a fertilized egg is implanted in the luminal epithelium on the cilia, providing a microenvironment essential for placentation.

In recent years, an attempt has been made to prepare a three dimensional cell structure similar to a tissue structure, called an organoid, in a living body, and to use the three dimensional cell structure as a tissue model in a living body. Also in the case of the endometrium, an example has been reported in which an endometrial organoid was prepared using endometrial epithelial cells or the like (Non-Patent Document 1).

### Citation List

### Non-Patent Literature

Non-Patent Document 1: Margherita Y. Turco et al., Long-term, hormone-responsive organoid cultures of human endometrium in a chemically defined medium. Nature Cell Biology volume 19, 568-577 (2017)

### Summary of Invention

### Technical Problem

The endometrial organoid of Non-Patent Document 1 has a simple spherical structure, which is different, as the spatial morphological structure, from that of the endometrium of a living body. Also, embryo implantation cannot be simulated because of the presence of an apical surface receiving an embryo within the structure.

Accordingly, an object of the present invention is to provide an endometrial model in which apical surfaces of endometrial epithelial cells are exposed, a method for preparing the endometrial model, and an endometrial implantation model using the endometrial model.

### Solution to Problem

The present invention includes the following aspects.
[1] An endometrial model including: a stromal extracellular matrix-containing gel; and endometrial epithelial cells, wherein at least some of the endometrial epithelial cells are exposed on a surface of the gel, and the at least some of the endometrial epithelial cells exposed on the surface of the gel continuously form a luminal structure and a non-luminal structure.
[2] The endometrial model according to [1], wherein the non-luminal structure is a flat membranous structure.
[3] The endometrial model according to [1] or [2], wherein the stromal extracellular matrix is type 1 collagen.
[4] The endometrial model according to any one of [1] to [3], wherein some of the endometrial epithelial cells are present inside the gel.
[5] A method for preparing an endometrial model, including: (a) allowing endometrial epithelial cells to be present inside a stromal extracellular matrix-containing gel; and (b) after the step (a), culturing the endometrial epithelial cells.
[6] The method for preparing an endometrial model according to [4], wherein the stromal extracellular matrix is type I collagen.
[7] An endometrial implantation model including: the endometrial model described in any one of [1] to [4]; and an embryonic cell.
[8] The endometrial implantation model according to [7], wherein the embryonic cells comprise at least one type of cells selected from the group consisting of trophoblast stem cells, cytotrophoblast cells, extravillous trophoblast cells, syncytiotrophoblast cells, pluripotent stem cells, and cells derived therefrom.
[9] The endometrial implantation model according to [7] or [8], wherein the embryonic cells form spheroids or organoids.

### Advantageous Effects of Invention

The present invention provides an endometrial model in which apical surfaces of endometrial epithelial cells are exposed, a method for preparing the endometrial model, and an endometrial implantation model using the endometrial model.

### Brief Description of Drawings

FIG. 1 is a schematic diagram showing an example of an endometrial model.
FIG. 2 is a schematic diagram showing an example of an endometrial implantation model.
FIG. 3 shows bright field phase contrast microscopy images of endometrial models prepared using Matrigel or a type 1 collagen gel.
FIG. 4 shows immunostaining images of endometrial models prepared using Matrigel or type 1 collagen gel.
FIG. 5 shows immunostaining images of an endometrial model prepared using type I collagen gel.
FIG. 6 shows images of endometrial epithelial cells plate-cultured with Matrigel or a type 1 collagen gel.
FIG. 7 shows an endometrial implantation model prepared by co-culturing an endometrial model prepared using type 1 collagen gel and a blastocyst-like structure derived from ES cells.

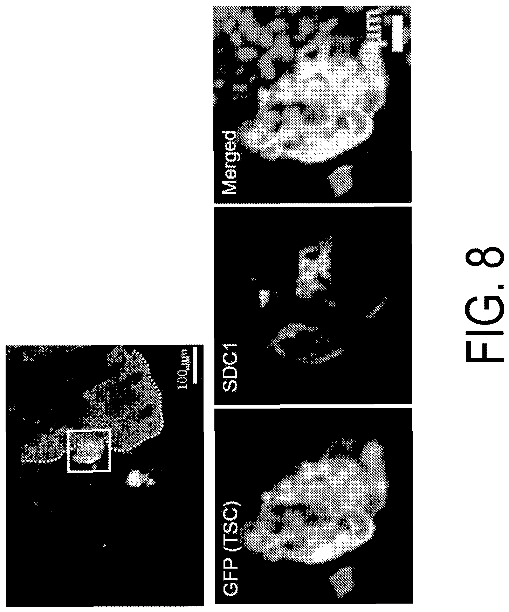

FIG. 8 shows an endometrial implantation model prepared by co-culturing an endometrial model prepared using type I collagen gel and a blastocyst-like structure derived from TS cells. The upper image is obtained by merging fluorescence microscopic images in which GFP, OCT4, SCD1, F-actin, and Hoechast are detected. The "Merged" among the lower images is an enlarged view of a boxed portion in the upper image. The "GFP (TSC)" among the lower images is a fluorescence microscope image in which GFP in the boxed portion in the upper image is detected. The "SDC1" among the lower images is a fluorescence microscopic image in which SDC1 of the boxed portion in the upper image is detected.

### Description of Embodiments

Hereinafter, embodiments of the present invention will be described in detail with reference to the accompanying drawings. In the drawings, the same or corresponding portions are denoted by the same or corresponding reference numerals, and redundant descriptions will be omitted. Dimensional ratios in the drawings are exaggerated for the sake of explanation and do not necessarily coincide with actual dimensional ratios.

### [Definition]

The term "comprise" means that components other than the component in question may be included. The term "consist of" means that no component other than the component in question is included. The term "consist essentially of" means that no component other than the component in question is included in an aspect in which a special function is exerted (such as an aspect in which the effect of the invention is completely lost). As used herein, references to "comprise" encompass embodiments that "consist of" and embodiments that "consist essentially of".

A "cell" as described herein may be isolated. The term "isolated" refers to a state of being separated from the natural state. The "cell" as described herein may be an isolated cell.

### [Endometrial Model]

A first aspect of the present disclosure is an endometrial model. In one embodiment, the endometrial model includes: a stromal extracellular matrix-containing gel; and endometrial epithelial cells. In one embodiment, at least some of the endometrial epithelial cells are exposed on a surface of the gel. In one embodiment, the at least some of the endometrial epithelial cells exposed on the surface of the gel continuously form a luminal structure and a non-luminal structure.

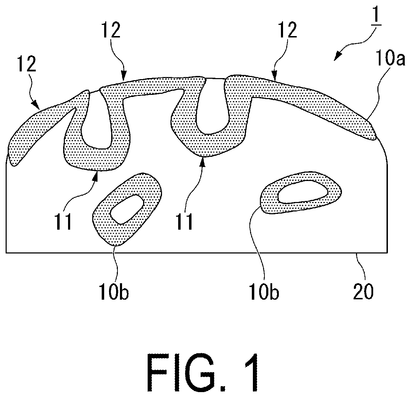

FIG. 1 is a schematic diagram showing an endometrial model according to one embodiment. An endometrial model 1 includes a stromal extracellular matrix-containing gel 20 and cell assemblies (10a, 10b) of endometrial epithelial cells. The cell assembly 10a is a cell assembly of endometrial epithelial cells exposed on the stromal extracellular matrix-containing gel 20. The cell assembly 10b is a cell assembly of endometrial epithelial cells present inside the stromal extracellular matrix-containing gel 20.

The cell assembly 10a is exposed on the surface of the stromal extracellular matrix-containing gel 20, and has a structure in which a luminal structure 11 and a non-luminal structure 12 are continuously formed. The term "luminal structure" refers to a structure in which an endometrial epithelial cell invaginates into the stromal extracellular matrix-containing gel 20. The term "non-luminal structure" refers to a structure that is not the luminal structure. Examples of the non-luminal structure include flat membranous structures. The structure "in which a luminal structure and a non-luminal structure are continuously formed" refers to a structure which includes at least one luminal structure and at least one non-luminal structure, and in which the luminal structure and the non-luminal structure are connected to each other.

The number of the luminal structures 11 included in the cell assembly 10a is not particularly limited. Depending on the size of the cell assembly 10a, the cell assembly 10a may have a plurality of luminal structures 11. When the cell assembly 10a has the plurality of luminal structures 11, two luminal structures 11 are connected by the non-luminal structure 12.

The endometrial epithelial cells forming the luminal structure 11 may express a progesterone receptor (PGR). The luminal structure 11 may have a function similar to that of mature endometrial glandular epithelium.

The stromal extracellular matrix-containing gel 20 contains a stromal extracellular matrix. The "stromal extracellular matrix" is an extracellular matrix predominantly present in the stroma. Examples of the stromal extracellular matrix include type 1 collagen, proteoglycans (versican, decorin, and the like), and fibronectin. Among them, the type I collagen is preferable as the stromal extracellular matrix. One type or two or more types of stromal extracellular matrix/matrices may be contained in the stromal extracellular matrix-containing gel 20.

The stromal extracellular matrix-containing gel 20 may contain any other extracellular matrix in addition to the stromal extracellular matrix. Examples of the other extracellular matrix include basement membrane extracellular matrices such as laminin, type IV collagen, heparan sulfate proteoglycan, and entactin; and cartilage extracellular matrices such as hyaluronic acid, type II collagen, and link protein. Commercially available products such as Matrigel (registered trademark) may also be used. When the stromal extracellular matrix-containing gel 20 contains any other extracellular matrix, the other extracellular matrix may be of one type or two or more types.

The stromal extracellular matrix-containing gel 20 preferably contains the stromal extracellular matrix gel in an amount of 50 vol% or more, 55 vol% or more, 60 vol% or more, 65 vol% or more, 70 vol% or more, 75 vol% or more, or 80 vol% or more with respect to a total gel volume. The stromal extracellular matrix-containing gel 20 may be 100 vol% of the stromal extracellular matrix gel.

### (Maintenance Medium for Endometrial Model)

The endometrial model 1 can be maintained in a medium or the like. The medium is not particularly limited as long as it is a medium capable of maintaining the endometrial model 1. Examples of the medium include media obtained by adding growth factors, ROCK inhibitors, GSK3β inhibitors, p38 MAPK inhibitors, maturation inducing factors and the like to a basal medium generally used for culturing animal cells.

### Basal Medium

Examples of the basal medium include Doulbecco's modified Eagle's Medium (DMEM) medium, DMEMIF12 medium, Advanced DMEM/F12 medium, IMDM medium, Medium 199 medium, Eagle's Minimum Essential Medium (EMEM) medium, αMEM medium, Ham's F12 medium, RPMT1640 medium, Fischer's medium, and mixed media thereof. For example, DMEM/F12 is a preferred basal medium.

The basal medium may contain serum (such as fetal bovine serum (FBS)) and/or a serum replacement, as necessary. Examples of the serum replacement include albumin, transferrin, sodium selenite, ITS-X (Invitrogen), Knockout Serum Replacement (KSR), N2 supplement (Invitrogen), B27 supplement (Invitrogen), fatty acids, insulin, collagen precursors, trace elements, 2-mercaptoethanol, and 3'-thiolglycerol. The basal medium may contain components such as lipids, amino acids, L-glutamine, Glutamax, non-essential amino acids, vitamins, growth factors, antibiotics, antioxidants, pyruvic acid, buffers, and inorganic salts, as necessary. These components can be used in an appropriate combination.

Examples of the basal medium include media obtained by adding bovine serum albumin (BSA), ITS-X, L-ascorbic acid, and antibiotics (penicillin, streptomycin, and the like) to the basal media as described above (for example, DMEM/F12). A specific example of the basal medium is a TS basal medium used in the Examples which will be described later.

### Growth Factor

Examples of the growth factor include, but are not particularly limited to, epidermal growth factor (EGF), fibroblast growth factor (FGF), and bone morphogenetic protein (BMP).

EGF binds to EGF receptors (EGFRs) present on the cell surface to induce EGF signaling and acts as a mitogen. An organism from which the EGF is derived is not particularly limited, but is preferably human EGF. Human FGF may be a recombinant produced by a non-human cell. A commercially available EGF can be used. A concentration of the EGF in the medium is not particularly limited, and can be, for example, from 5 to 200 ng/mL. The concentration of the EGF in the medium is preferably from 10 to 150 ng/mL, more preferably from 10 to 100 ng/mL, further preferably from 10 to 80 ng/mL, and particularly preferably from 10 to 60 ng/mL.

The FGF has a high affinity for heparan sulfate proteoglycans and forms complexes with cell surface FGF receptors (FGFRs), together with heparan sulfate proteoglycans, to induce FGF signaling, and acts as a mitogen. An organism from which the FGF is derived is not particularly limited, but is preferably FGF. The human FGF may be a recombinant produced by a non-human cell. As the FGF, FGF2 (also referred to as bFGF) is preferable. A commercially available FGF can be used. A concentration of the FGF (e.g., FGF2) in the medium is not particularly limited, and can be, for example, from 5 to 200 ng/mL. The concentration of the FGF (e.g., FGF2) in the medium is preferably from 10 to 150 ng/mL, more preferably from 20 to 100 ng/mL, further preferably from 30 to 80 ng/mL, and particularly preferably from 40 to 60 ng/mL.

When the medium contains FGF, the medium may contain heparin. The heparin has the effect for promoting the activity of the FGF. The heparin is preferably in the form of a salt. Examples of the salt of the heparin include salts with alkali metals such as lithium, sodium, and potassium; salts with alkaline earth metals such as calcium, barium, and magnesium; salts with metals such as aluminum, zinc, copper, and iron; ammonium salts; salts with organic bases; and salts with amino acids. A commercially available heparin can be used. A concentration of the heparin in the medium is not particularly limited, and can be, for example, from 0.001 to 10 µg/mL. The concentration of the heparin in the medium is preferably from 0.005 to 5 µg/mL, more preferably from 0.01 to 3 µg/mL, further preferably from 0.05 to 1 µg/mL, and particularly preferably from 0.07 to 0.5 µg/mL.

The BMP is a protein belonging to the transforming growth factor β(TGFβ) superfamily, and controls induction of cell death, cell differentiation, and the like. An organism from which the BMP is derived is not particularly limited, but is preferably human BMP. The human BMP may be a recombinant produced by a non-human cell. BMP4 is preferred as the BMP. A commercially available BMP can be used. A concentration of the BMP (e.g., BMP4) in the medium is not particularly limited, and can be, for example, from 5 to 200 ng/mL. The concentration of the BMP (e.g., BMP4) in the medium is preferably from 10 to 150 ng/mL, more preferably from 20 to 100 ng/mL, further preferably from 30 to 80 ng/mL, and particularly preferably from 40 to 60 ng/mL.

### ROCK Inhibitor

A ROCK (Rho associated coiled-coil containing protein kinase: Rho-associated kinase) inhibitor is a substance that inhibits the function of Rho-associated kinase. Examples of the ROCK inhibitor include trans-N-(4-pyridyl)-4-(1-aminoethyl)-cyclohexanecarboxamide, 1-(5-isoquinolinylsulfonyl)homopiperazine), and salts thereof. Further examples of the inhibitor include small molecule inhibitors such as Fasudil/HA1077, H-1152, and Wf-536, and derivatives thereof. The ROCK inhibitor may be an antisense nucleic acid against ROCK, siRNA, a dominant negative mutant, an expression vector thereof, or the like.

Examples of commercially available trans-N-(4-pyridyl)-4-(1-aminoethyl)-cyclohexanecarboxamide or salts thereof include Y27632 ((R)-(+)-trans-N-(4-pyridyl)-4-(1-aminoethyl)-cyclohexanecarboxamide•2HCl•H₂O). For the ROCK inhibitor, one type may be used alone, or two or more types may be used in combination.

Preferably, the Y27632 is used as the ROCK inhibitor.

A concentration of the ROCK inhibitor in the medium is not particularly limited, and can be, for example, from 0.1 to 50 µM, preferably from 1 to 20 µM, more preferably from 1 to 15 µM, and further preferably from 3 to 12 µM.

### GSK3β Inhibitor

The GSK (Glycogen Synthase Kinase) 3β inhibitor is a substance that inhibits the function of GSK3β, for example, kinase activity (e.g., ability to phosphorylate β-catenin). Examples of the GSK3β inhibitor include small molecule inhibitors such as 6-[[2-[[4-(2,4-dichlorophenyl)-5-(4-methyl-1H-imidazol-2-yl)-2-pyrimidinyl]amino]ethyl]amino]nicotinonitrile, Kenpaullone, 1-azakenpaullone, CHIR98014, AR-A014418, CT99021, CT20026, SB216763, AR-A014418, lithium, SB415286, TDZD-8, BIO, BIO-acetoxime, (5-methyl-1H-pyrazol-3-yl)-(2-phenylquinazolin-4-yl)amine, pyridocarbazole-cyclopentadienyl ruthenium complexes, TDZD-8 4-benzyl-2-methyl-1,2,4-thiadiazolidine-3, 5-dione, 2-thio(3-iodobenzyl)-5-(1-pyridyl)-[1,3,4]-oxadiazole, OTDZT, alpha-4-dibromoacetophenone, AR-AO 144-18, 3-(1-(3-hydroxypropyl)-1H-pyrrolo[2,3-b]pyridin-3-yl]-4-pyrazin-2-yl-pyrrole-2,5-dione; TWSl19 pyrrolopyrimidine compounds, L803 H-KEAPPAPPQSpP-NH2 or its myristoylated form; 2-chloro-1-(4,5-dibromo-thiophen-2-yl)-ethanone, SB216763, and SB415286. The GSK3β inhibitor may be an antisense nucleic acid against GSK3β, siRNA, a dominant negative mutant, an expression vector thereof, or the like. Examples of commercially available products of 6-[[2-[[4-(2,4-dichlorophenyl)-5-(4-methyl-1H-imidazol-2-yl)-2-pyrimidinyl]amino]ethyl]amino]nicotinonitrile include CHIR99021. For the GSK3β inhibitor, one type may be used alone, or two or more types may be used in combination.

Preferably, the CHIR99021 is used as the GSK3β inhibitor.
A concentration of the GSK3β inhibitor in the medium is not particularly limited, and can be, for example, from 0.1 to 20 µM, preferably from 0.2 to 10 µM, more preferably from 0.5 to 5 µM, and further preferably from 0.5 to 3 µM.

### p38 MAPK inhibitor

The p38 MAPK inhibitor is a substance that inhibits the function of p38 MAPK (p38 mitogen-activated protein kinase). Examples of the p38 MAPK inhibitor include SB202190 (4-(4-fluorophenyl)-2-(4-hydroxyphenyl)-5-(4-pyridyl)-1H-imidazole), SB203580 (4-[4-(4-fluorophenyl)-2-[4-(methylsulfinyl)phenyl]-1H-inudazol-5-yl]pyridine), VX702 (6-(N-carbamoyl-2,6-difluoroanilino)-2-(2,4-difluorophenyl)pyridine-3-carboxamide), VX745 (5-(2,6-dichlorophenyl)-2-[2,4-difluorophenyl)thio]-6H-pyrimido[1,6-b]pyridazin-6-one), PD169316 (4-(4-fluorophenyl)-2-(4-nitrophenyl)-5-(4-pyridyl)-1H-imidazole), RO4402257 (6-(2,4-difluorophenoxy)-2-{[3-hydroxy-1-(2-hydroxyethyl)propyl]amino)-8-methylpyrido[2,3-D]pyrimidin-7(8h)-one), and BIRB796 (1-[5-tert-butyl-2-(4-methylphenyl)pyrazol-3-yl]-3-[4-(2-morpholine-4-irethoxy)naphthalen-1-yl]urea). The p38 MAPK inhibitor may be an antisense nucleic acid against p38 MAPK, siRNA, a dominant negative mutant, an expression vector thereof, or the like. For the p38 MAPK inhibitor, one type may be used alone, or two or more types may be used in combination.

Preferably, the SB202190 is used as the p38 MAPK inhibitor.

A concentration of the p38 MAPK inhibitor in the medium is not particularly limited, and can be, for example, from 0.1 to 20 µM, preferably from 0.2 to 10 µM, more preferably from 0.5 to 5 µM, and further preferably from 0.5 to 3 µM.

### Maturation Inducing Factor

The maturation inducing factor is a factor that induces maturation of endometrial cells. The maturation inducing factor is not particularly limited as long as it is a factor capable of inducing maturation of endometrial cells. Examples of the maturation inducing factor include estrogen receptor ligand, progesterone receptor ligand, cyclic AMP (cAMP) or a derivative thereof, prolactin, placental lactogen, and chorionic gonadotropin.

The estrogen receptor ligand is a substance that binds to an estrogen receptor. Examples of the estrogen receptor ligand include estrone, estradiol, and estriol. Examples of the estradiol include β-estradiol and 17β-estradiol. For the estrogen receptor ligand, one type may be used alone, or two or more types may be used in combination. Preferably, the estradiol is used as the estrogen receptor ligand.

A concentration of the estrogen receptor ligand in the medium is not particularly limited, and can be, for example, from 0.1 to 50 µM, preferably from 0.5 to 30 µM, more preferably from 1 to 20 µM, and further preferably from 5 to 15 µM.

The progesterone receptor ligand is a substance that binds to a progesterone receptor. Examples of the progesterone receptor ligand include progesterone and medroxyprogesterone acetate. For the progesterone receptor ligand, one type may be used alone, or two or more types may be used in combination. Preferably, the medroxyprogesterone acetate is used as the progesterone receptor ligand.

A concentration of progesterone or a derivative thereof in the medium is not particularly limited, and can be, for example, from 0.1 to 20 µM, preferably from 0.2 to 10 µM, more preferably from 0.5 to 5 µM, and further preferably from 0.5 to 3 µM.

Examples of the cAMP or derivative thereof include 8-bromoadenosine- 3-5- cyclic monophosphate (8-Br-cAMP), dibutyryl-cAMP, and 8-CPT-2-Me-cAMP sodium salt (8-(4-chlorophenylthio)-2'-O-methyladenosine-3', 5'-cyclic monophosphate sodium salt). For the cAMP or derivative thereof, one type may be used alone, or two or more types may be used in combination. Preferably, the 8-Br-cAMP is used the cAMP or derivative thereof.

A concentration of the cAMP or derivative thereof in the medium is not particularly limited, and can be, for example, from 0.1 to 200 µM, preferably from 1 to 150 µM, more preferably from 10 to 100 µM, and further preferably from 20 to 80 µM.

Prolactin is a hormone secreted by prolactin-secreting cells of the anterior pituitary. An organism from which prolactin is derived is not particularly limited, but is preferably human prolactin. The human prolactin may be a recombinant produced by a non-human cell. A commercially available prolactin can be used. A concentration of the prolactin in the medium is not particularly limited, and can be, for example, from 5 to 200 ng/mL. The concentration of the prolactin in the medium is preferably from 10 to 150 ng/mL, more preferably from 10 to 100 ng/mL, further preferably from 10 to 80 ng/mL, and particularly preferably from 10 to 60 ng/mL.

The placental lactogen is a peptide hormone secreted from the placenta. An organism from which the placental lactogen is derived is not particularly limited, but is preferably human placental lactogen. The human placental lactogen may be a recombinant produced by a non-human cell. A commercially available placental lactogen can be used. A concentration of the placental lactogen in the medium is not particularly limited, and can be, for example, from 5 to 200 ng/mL. The concentration of the placental lactogen in the medium is preferably from 10 to 150 ng/mL, more preferably from 10 to 100 ng/mL, further preferably from 10 to 80 ng/mL, and particularly preferably from 10 to 60 ng/mL.

A chorionic gonatodropin is a peptide hormone secreted from syncytiotrophoblast. An organism from which the chorionic gonatodropin is derived is not particularly limited, but is preferably human chorionic gonatodropin. The human chorionic gonatodropin may be a recombinant produced by a non-human cell. A commercially available chorionic gonatodropin can be used. The concentration of the chorionic gonatodropin in the medium is not particularly limited, and can be, for example, from 0.01 to 100 µg/mL. The concentration of the chorionic gonatodropin in the medium is preferably from 0.05 to 50 µg/mL, more preferably from 0.1 to 20 µg/mL, further preferably from 0.2 to 10 µg/mL, and particularly preferably from 0.5 to 5 µg/mL.

Examples of a maintenance medium for the endometrial model include media obtained by adding growth factors (EGF and the like), ROCK inhibitors (Y27632 and the like), GSK3β inhibitors (CH1R99021 and the like), p38 MAPK inhibitors (SB202190 and the like), and the like to a basal medium for animals cells (for example, TS basal medium and the like). Specific examples of such a medium include an ECSY medium used in the Examples which will be described later.

Examples of the maintenance medium for the endometrial model include media obtained by adding growth factors (EGF and the like), ROCK inhibitors (Y27632 and the like), GSK3β inhibitors (CH1R99021 and the like), p38 MAPK inhibitors (SB202190 and the like), maturation inducing factors (estradiol, medroxyprogesterone acetate, 8-Br-cAMP and the like), and the like to a basal medium for animal cells (for example, TS basal medium and the like). Specific examples of such a medium include an ECSY + EPC medium used in the Examples which will be described later.

When further maturation of the endometrial epithelial cells in the endometrial model is induced, further maturation inducing factors (prolactin, placental lactogen, chorionic gonadotropin, etc.) may be added to the maintenance medium as described above. Examples of the medium include media obtained by adding growth factors (EGF and the like), ROCK inhibitors (Y27632 and the like), GSK3β inhibitors (CHIR99021 and the like), p38 MAPK inhibitors (SB202190 and the like), maturation inducing factors (estradiol, medroxyprogesterone acetate, 8-Br-cAMP, prolactin, placental lactogen, chorionic gonadotropin and the like) and the like to a basal medium for animal cells (for example, TS basal medium and the like). Specific examples of such a medium include media (for example, ECSY + EPC + PLG medium) obtained by adding human prolactin (for example, 20 ng/mL), human placental lactogen (for example, 20 ng/mL), and human chorionic gonadotropin (for example, 1 µg/mL) to an ECSY + EPC medium used in the Examples which will be described later.

The endometrial model of the present aspect has a structure in which endometrial epithelial cells are exposed on the surface of the gel. In addition, it has a luminal structure similar to that of endometrial glandular epithelium. These structural features are similar to those of the endometrium of a living body. Therefore, the endometrial model of the present aspect can be applied to drug screening and drug evaluation for diseases related to the endometrium (endometriosis, endometrial cancer, and the like). Also, since the apical surfaces of the endometrial epithelial cells are exposed, implantation can be simulated. Therefore, it can be applied to the elucidation of the implantation mechanism or implantation failure, screening of implantation failure therapeutic agents, and evaluation of implantation failure therapeutic agents.

### [Method for Preparing Endometrial Model]

A second aspect of the present disclosure is a method for preparing an endometrial model. The method according to the present aspect includes steps of: (a) allowing endometrial epithelial cells to be present inside a stromal extracellular matrix-containing gel; and (b) culturing the endometrial epithelial cells.

### Step (a)

In step (a), the endometrial epithelial cells are allowed to be present inside the stromal extracellular matrix-containing gel.

### (Endometrial Epithelial Cell)

The endometrial epithelial cells may be isolated from endometrial epithelial tissue, or a culture of the endometrial epithelial cells may be used. As a method for isolating the endometrial epithelial cells from the endometrial epithelial tissue from the endometrial tissue, a known method can be used. As the endometrial epithelial tissue, a decidua may be used.

The endometrial epithelial cells can be isolated, for example, by separating the cells using mechanical and/or enzymatic treatment of the endometrial epithelial tissue as appropriate. Examples of the mechanical treatment include cutting with a scalpel. Examples of the enzymatic treatment include treatment with a protease having extracellular matrix-degrading activity (e.g., Dispase, collagenase, etc.). After mechanical and/or enzymatic treatment of the endometrial epithelial tissue, the endometrial epithelial cells can be recovered using a strainer or filter or the like. The recovered endometrial epithelial cells may be appropriately washed with a medium or the like.

The isolated endometrial epithelial cells may be prepared by appropriately adding ice-cooled Matrigel or the like and culturing the cells. Examples of a culture temperature in Matrigel include from 30 to 40°C (for example, 37°C). A culture time is not particularly limited and may be, for example, 5 minutes or more, 10 minutes or more, or 15 minutes or more. An upper limit of the culture time is not particularly limited and may be, for example, 60 minutes or less, 50 minutes or less, 40 minutes or less, 30 minutes or less, or 20 minutes or less.

The prepared endometrial epithelial cells can be maintained in an ECSY medium or the like.

### (Stromal Extracellular Matrix-Containing Gel)

As the stromal extracellular matrix-containing gel, the same gel as described above can be used. The stromal extracellular matrix-containing gel preferably contains type I collagen as the stromal extracellular matrix.

### (Method for Seeding Endometrial Epithelial Cell)

A method for allowing the endometrial epithelial cells to be present inside the stromal extracellular matrix-containing gel is not particularly limited. For example, the endometrial epithelial cells may be recovered from a culture solution of the cells by centrifugation or the like, and mixed with the stromal extracellular matrix-containing gel. Alternatively, the endometrial epithelial cells may be suspended in an appropriate medium to prepare a cell suspension, and the cell suspension may be injected into the stromal extracellular matrix-containing gel.

The number of the endometrial epithelial cells to be seeded in the stromal extracellular matrix-containing gel is not particularly limited. Examples of the number of the cells to be seeded is, for example, 1 or more, 10 or more, 10² or more, 10³ or more, 10⁴ or more, 10⁵ or more, 10⁶ or more, 10⁷ or more, 10⁸ or more, 10⁹ or more, or 10¹⁰ or more. An upper limit of the number of the cells to be seeded is not particularly limited. For example, the upper limit is 10²⁰ or less, 10¹⁵ or less, 10¹⁴ or less, 10¹³ or less, or 10¹² or less. The lower limit values and the upper limit values can be appropriately combined.

### (Step (b))

In step (b), the endometrial epithelial cells are cultured after the step (a).

The culture is started in a state in which the endometrial epithelial cells are present inside the stromal extracellular matrix-containing gel. The culture may be carried out by adding an appropriate medium to the stromal extracellular matrix-containing gel containing the endometrial epithelial cells. Examples of a culture vessel include, but are not limited to, a non-treated well plate and a non-treated dish.

As the culture medium, those listed above can be used. During the culture, it is preferable to appropriately change the medium. A frequency of the medium change is not particularly limited, and may be, for example, 2 to 3 times per week.

In order to promote maturation of the endometrial epithelial cells, a maturation inducing factor may be appropriately added to the medium. For example, at an initial stage of the culture, the cells may be cultured in a maturation inducing factor-free medium. Then, the medium may be replaced with a maturation inducing factor-containing medium, and further culture may be performed.

Examples of a period of the culture in a maturation factor-free medium (e.g., ECSY medium) include 1 day or more, 2 days or more, 3 days or more, 4 days or more, 5 days or more, 6 days or more, and 7 days or more. An upper limit of the period of the culture in the maturation factor-free medium (e.g., ECSY medium) is not particularly limited, and may be, for example, 30 days or less, 25 days or less, 20 days or less, 15 days or less, 12 days or less, or 10 days or less. The lower limit values and the upper limit values can be appropriately combined.

The period of the culture in a maturation factor-containing medium (e.g., ECSY + EPC medium) is, for example, 1 day or more, 2 days or more, 3 days or more, 4 days or more, 5 days or more, 6 days or more, or 7 days or more. An upper limit of the period of the culture in the maturation factor-containing medium (for example, ECSY medium) is not particularly limited, and may be, for example, 30 days or less, 25 days or less, 20 days or less, 15 days or less, 12 days or less, or 10 days or less. The lower limit values and the upper limit values can be appropriately combined.

In order to further promote maturation of the endometrial epithelial cells, a medium containing, as the maturation inducing factors, three or more, four or more, or five or more maturation inducing additive factors selected from the group consisting of an estrogen receptor ligand, a progesterone receptor ligand, cAMP or a derivative thereof, prolactin, placental lactogen, and chorionic gonadotropin may be used. For example, a medium containing all of an estrogen receptor ligand, a progesterone receptor ligand, cAMP or a derivative thereof, prolactin, placental lactogen, and chorionic gonadotropin (e.g., ECSY + EPC + PLG medium) may be used.

During the culture, the endometrial epithelial cells inside the stromal extracellular matrix-containing gel move through the gel and some of the cells reach the gel surface. The cells that have reached the gel surface form a structure in which a luminal structure and a non-luminal structure are continuous on the gel surface. The culture in the maturation inducing factor-containing medium can be continued until the endometrial epithelial cells form the structure.

Culture conditions can be those generally used for culturing animal cells. For example, the culture temperature can be from 32 to 40°C (preferably from 35 to 38°C, typically 37°C), and a CO₂ concentration can be 2 to 5% (preferably 5%).

The endometrial model according to the first aspect can be prepared by the method of the present aspect.

### [Endometrial Implantation Model]

A third aspect of the disclosure is an endometrial implantation model. The endometrial implantation model according to the present aspect includes the endometrial model according to the first aspect and an embryonic cell.

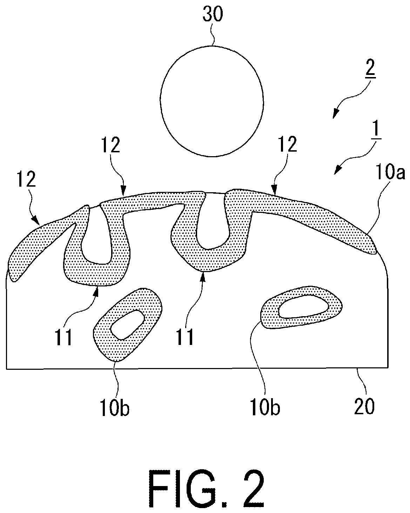

FIG. 2 is a schematic diagram showing an endometrial implantation model according to one embodiment. An endometrial implantation model 2 includes the endometrial model 1 and an embryonic cell 30. The embryonic cell 30 may or may not be implanted on the apical surface of the endometrial model 1.

### (Embryonic cell)

The term "embryonic cell" refers to a cell capable of simulating implantation into the endometrium.

A fertilized egg moves through the oviduct and reaches the uterus. The fertilized egg is cleaved while moving through the oviduct, and becomes a blastocyst via a 2-cell stage embryo, a 4-cell stage embryo, an 8-cell stage embryo, and a morula. When the blastocyst that has reached the uterus becomes an expanded blastocyst, the zona pellucida becomes thin and breaks, and thus the expanded blastocyst becomes a post-hatching blastocyst. The post-hatching blastocyst settles against the endometrium. The blastocyst is composed of an inner cell mass, a blastocyst cavity and a trophoblast, and trophoblast cells invade the endometrium to establish implantation. The embryonic cell is typically a cell constituting an embryo, and may be any of a fertilized egg, a 2-cell stage embryo, a 4-cell stage embryo, an 8-cell stage embryo, a morula, and a blastocyst. The embryonic cell may be a cell isolated from an embryo as described above, or a cell derived from the isolated cell. The embryonic cell may be a pluripotent stem cell or a cell derived from a pluripotent stem cell. The embryonic cell may be a cell isolated from a placenta or a cell derived from the cell.

Examples of the embryonic cells include trophoblast stem cells, cytotrophoblast cells, extravillous trophoblast cells, syncytiotrophoblast cells, pluripotent stem cells, and cells derived therefrom.

The trophoblast stem cells (TS cells) may be derived from blastocysts, derived from cytotrophoblast cells (CT cells), or derived from pluripotent stem cells.

The TS cells can be derived from blastocysts by known methods. For example, placental tissue is subjected to mechanical and/or enzymatic treatment as appropriate to separate cells. After that, the cells are cultured in a medium for induction into TS cells, and then the TS cells can be established using expression of a TS cell marker (GATA2 positive, GATA3 positive, TFAP2 positive, ELF5 positive, ZNF750 positive, CDX2 negative, or the like) as an indicator.

As a method for inducing TS cells from CT cells, for example, the method described in JP 6400832 B can be used.

As a method for inducing TS cells from pluripotent stem cells, for example, the method described in WO 2020/250438 and the like can be used.

For example, the CT cells can be those isolated from a placenta. The CT cells can be isolated from a placenta, for example, by mechanically and/or enzymatically treating placental tissue as appropriate to separate cells, and using the expression of a CT cell marker (CD49f positive, E-cadherin positive, or the like) as an indicator to isolate the CT cells (JP 6400832 B; Haider, S., et al., Stem Cell Reports 11, 537 to 551 (2018)).

The syncytiotrophoblast cells (ST cells) may be isolated from a placenta or may be derived from the TS cells. The ST cells can be isolated from a placenta, for example, by mechanically and/or enzymatically treating placental tissue as appropriate to separate cells, and using expression of a ST cell marker (Syndecan1 (SDC1) positive, human chorionic gonadotropin (hCG) positive, or the like) as an indicator to isolate the ST cells.

Examples of a method for inducing ST cells from TS cells include the method described in WO 2020/250438.

The extravillous trophoblast cells (EVT cells) may be isolated from a placenta or may be derived from TS cells. In the isolation of the EVT cells from a placenta, for example, the cells are separated by mechanically and/or enzymatically treating placental tissue as appropriate, and the EVT cells can be isolated using expression of an EVT cell marker (HLA-G positive or the like) as an indicator.

Examples of a method for inducing EVT cells from TS cells include the method described in WO 2020/250438.

The pluripotent stem cells are cells having pluripotency, and may be embryonic stem cells (ES cells), may be induced pluripotent stem cells (iPS cells), or may be cells having pluripotency induced from these stem cells. A method for producing pluripotent stem cells is not particularly limited, and a known method can be used. Cell lines of ES cells and iPS cells are also available from cell banks such as the Riken BioResource Research Center (RIKEN BRC). The pluripotent stem cells are preferably ES cells or iPS cells, and more preferably ES cells.

The embryonic cells may form spheroids or organoids. Formation of spheroids or organoids from the embryonic cells can be carried out by known methods. For example, spheroids or organoids of the embryonic cells can be prepared by subjecting embryonic cells to suspension culture using a medium as described above (e.g., a medium obtained by adding growth factors, ROCK inhibitors, GSK β inhibitors, and p38 MAPK inhibitors to a basal medium).

The spheroids or organoids of the embryonic cells may be cell aggregates derived from TS cells. For example, the TS cells can be cultured in suspension in a TS medium (Okae et al Cell Stem Cell 2018) to induce the cell aggregates. The cell aggregate may contain one or more types of cells selected from the group consisting of TS cells, CT cells, ST cells, and EVT cells, or may contain one or more types of cells selected from the group consisting of TS cells, CT cells, and ST cells.

The spheroids or organoids of the embryonic cells may be blastocyst-like structures. The blastocyst-like structure is a cell structure having a structure similar to that of the blastocyst derived from pluripotent cells such as pluripotent stem cells. The blastocyst-like structure can be derived by known methods (e.g., Yu L, et al., Nature. 2021 Mar; 591 (7851): 620-626). For example, pluripotent stem cells can be cultured using a 5i/L/A medium, then cultured in a HDM medium, and then cultured in a TDM medium to obtain the blastocyst-like structure. As the pluripotent stem cell, a naive type or a primed type may be used, but a naive type is preferably used.

### (Preparation of Endometrial Implantation Model)

The endometrial implantation model can be prepared by co-culturing the endometrial model and embryonic cells. A medium used in the co-culture is not particularly limited, and examples thereof include IVC1 medium and IVC2 medium used in the Examples, in addition to the above-described media.

Examples of the medium for the co-culture include media (for example, ECSY + EPC medium) obtained by adding growth factors (EGF and the like), ROCK inhibitors (Y27632 and the like), GSK3β inhibitors (CHIR99021 and the like), p38 MAPK inhibitors (SB202190 and the like), maturation inducing factors (estradiol, medroxyprogesterone acetate, 8-Br-cAMP and the like), and the like to a basal medium for animal cells (for example, TS basal medium).

Alternatively, a medium further added with a maturation inducing factor such as prolactin, placental lactogen, or chorionic gonadotropin may be used as the medium for the co-culture. Examples of the medium include media (for example, ECSY + EPC + PLG medium) obtained by adding growth factors (EGF and the like), ROCK inhibitors (Y27632 and the like), GSK3β inhibitors (CHIR99021 and the like), p38 MAPK inhibitors (SB202190 and the like), maturation inducing factors (estradiol, medroxyprogesterone acetate, 8-Br-cAMP, prolactin, placental lactogen, chorionic gonadotropin and the like) and the like to a basal medium for animal cells (for example, TS basal medium and the like).

Alternatively, examples of the medium for the co-culture include media (for example, 1VC1 medium, IVC2 medium, and the like) obtained by adding serums (FBS and the like), serum substitutes (ITS-X, KSR and the like), amino acids (L-glutamine, N-acetyl-L-cysteine and the like), organic acids (pyruvic acid, lactic acid and the like), maturation inducing factors (estrogen receptor ligand, progesterone receptor ligand and the like), and the like to a basal medium (e.g., DMEM/F12 medium, Advanced DMEM/F12 medium, and the like). For example, co-culture may be performed in an IVC1 medium for a predetermined period from the start of the co-culture (for example, from half a day to three days, from half a day to two days, from half a day to one day, or the like), and then co-culture may be performed in an IVC2 medium.

Culture conditions can be those generally used for culturing animal cells. For example, the culture temperature can be from 32 to 40°C (preferably from 35 to 38°C, typically 37°C), and a CO₂ concentration can be 2 to 5% (preferably 5%).

When the spheroids or organoids of the embryonic cells are co-cultured with the endometrial model, the spheroids or organoids of the embryonic cells can be implanted on the apical surface of the endometrial model. In the implanted spheroids or organoids, the cells at an implantation site may have differentiated into SDC1-positive cells (e.g. ST cells).

Since the endometrial implantation model according to the present aspect includes the endometrial model of the first aspect and embryonic cells, implantation into the endometrium can be simulated. Therefore, the endometrial implantation model according to the present aspect can be applied to elucidation of the mechanism of implantation or implantation failure, screening of an implantation failure drug, and the like.

### Examples

The present invention will be described with reference to the following examples, but is not limited to the examples.

### Medium

### (TS Basal Medium)

The following components were added to a DMEM/F12 medium (FUJIFILM Wako) to prepare a TS basal medium. The concentrations shown below are the final concentrations of the components in the TS basal medium.
Bovine serum albumin (BSA) (FUJIFILM Wako) 0.15%
Penicillin 5000 units/mL.
Streptomycin (Thermo Fisher Scientific) 5000 µg/mL
1TS-X (FUJIFILM Wako) 1%
KSR (Thermo Fisher Scientific) 1%
L-ascorbic acid (FUJIFILM Wako) 0.2 mM

### (ECSY Medium)

An ECSY medium was prepared by adding the following additive factors to the TS basal medium. The concentrations shown below are the final concentrations of the components in the ECSY medium.
Human EGF, recombinant (FUJIFILM Wako) 50 ng/mL
CHIR99021 (FUJIFILM Wako) 2 µM
SB202190 (FUJIFILM Wako) 2 µM
Y27632 (FUJIFILM Wako) 10 µM

### (ECSY + EPC Medium)

The following maturation inducing additive factors were added to the ECSY medium to prepare an ECSY + EPC medium. The concentrations shown below are the final concentrations of the components in the ECSY + EPC medium.
Estradiol (SIGMA) 10 µM
Medroxyprogesterone acetate (MPA) (FUJIFILM Wako) 2 µM
8-Bromo-cAMP (SIGMA) 50 µM

### (ECSY + EPC + PLG Medium)

An ECSY + EPC + PLG medium was prepared by adding the following maturation inducing additive factors to the ECSY + EPC medium. The concentrations shown below are the final concentrations of the components in the ECSY + EPC + PLG medium.
Human prolactin, recombinant (Peprotech) 20 ng/mL
Human placental lactogen (hPL) (R & D systems) 20 ng/mL
Human chorionic gonadotropin (hCG) (SIGMA) 1 µg/mL

### (5i/L/A Medium)

A 5i/L/A medium was prepared by mixing the following components (heunissen, T. W. et al. Cell Stem Cell 15, 471-487 (2014)). The concentrations shown below are final concentrations of the components in the 5i/L/A medium.
DMEM/F12 medium (Invitrogen) 250 mL
Neurobasal medium (Invitrogen) 250 mL
N2 supplement (Invitrogen) 5 mL
B27 supplement (Invitrogen) 10 mL
1 × GlutaMAX (Gibco)
1 × Non-essential amino acids (Gibco)
β-mercaptoethanol (Gibco) 0.1 mM
Penicillin-Streptomycin (Gibco) 0.5%
Bovine serum albumin (BSA, Sigma) 50 mg/mL
PD0325901 (Stemgent) 1 µM
1M-12 (Enzo) 0.5 µM or 1 µM
SB590885 (R & D systems) 0.5 µM
WH-4-023 (A Chemtek) 1 µM
Recombinant human LIF (Peprotech) 20 ng/mL
Activin A (Peprotech) 10 ng/mL

### (HDM Medium)

A HDM medium was prepared by adding the following components to a 1:1 (v/v) mixed medium of DMEM/F12 medium and Neurobasal medium. The concentrations indicated below are final concentrations of the components in the HDM medium.
1 × N2 supplement
1 × B27 supplement
1 × GlutaMAX
1 × Non-essential amino acids
β-mercaptoethanol 0.1 mM
Penicillin-Streptomycin 0.5%
bFGF (Peprotech) 20 ng/mL
Activin A (Peprotech) 20 ng/mL
CHIR99021 3 µM

### (TDM Medium)

A TDM medium was prepared by adding the following components to a 1:1 (v/v) mixed medium of DMEM/F12 medium and Neurobasal medium. The concentrations given below are final concentrations of the components in the TDM medium.
0.5 × N2 supplement
0.5 × B27 supplement
ITS-X 0.5%
0.5 × GlutaMAX
0.5 × Non-essential amino acids
β-mercaptoethanol 0.1 mM
Knockout Serum Replacement (KSR, Gibco) 0.5% (v/v)
FBS 0.1%
BSA 50 mg/mL
Penicillin-Streptomycin 0.5%
PD0325901 1 µM
A83-01 0.5 µM
SB590885 0.25 µM
WH-4-023 0.5 µM
IM-12 0.25 µM
CH1R99021 1 µM
SB431542 0.5 µM
Recombinant human LIF 10 ng/mL
EGF 25 ng/mL
L-ascorbic acid 0.75 µg/mL
VPA 0.4 mM

### (IVC1 Medium)

An IVC1 medium was prepared by adding the following components to an Advanced DMEMIF12 medium (Gibco). The concentrations shown below are final concentrations of the components in the IVC1 medium.
FBS 20% (v/v)
L-glutamine (Gibco) 2 mM
1 × ITS-X
β-oestradiol (Sigma) 8 nM
Progesterone (Sigma) 200 ng/mL
N-acetyl-L-cysteine (Sigma) 25 µM
Sodium Lactate (Sigma) 0.22% (v/v)
Sodium Pyruvate (Sigma) 1 mM
Y27632 10 µM

### (1VC2 Medium)

An IVC2 medium was prepared by adding the following components to an Advanced DMEM/F12 medium. The concentrations shown below are final concentrations of the components in the IVC2 medium.
KSR 30% (v/v)
L-glutamine 2 mM
1 × 1TS-X
β-oestradiol 8 nM
Progesterone 200 ng/mL
N-acetyl-L-cysteine 25 µM
Sodium Lactate 0.22% (v/v)
Sodium Pyruvate 1 mM
Y27632 10 µM

### (TS Medium)

A TS medium was prepared by adding the following components to the TS basal medium. The concentrations shown below are the final concentrations of the components in the TS medium.
Y27632 (FUJIFILM Wako) 2.5 µM
EGF (FUJIFILM Wako) 25 ng/mL
VPA (FUJIFILM Wako) 0.8 mM
A83-01 (FUJIF`ILM Wako) 5 µM
CHIR99021 (FUJIFILM Wako) 2 µM

### Isolation of Endometrial Epithelial Cell

Endometrial epithelial cells were isolated by the following procedure.
1. A decidua was manually isolated using tweezers under a stereomicroscope.
2. The decidua was washed with a washing medium (RPM1640).
3. The decidua was transferred to a new dish and cut into a size of from 0.5 to 1 mm³ with a scalpel.
4. Enzymes (Dispase/collagenase) were added, and they were reacted at 37°C for from 1 to 2 hours with shaking.
5. A medium was added for neutralization.
6. The neutralized product was passed through a 100 µM filter from one to several times to capture the cells on the filter.

### Preparation of Endometrial Epithelial Cell

Endometrial epithelial cells were prepared by the following procedure.
1. The endometrial epithelial cells captured by the 100 µM filter in item 6 of isolation of Endometrial Epithelial Cell> were recovered by inverting the 100 µM filter and washing it with a washing medium (RPM1640).
2. Centrifugation was performed at 500 g for 5 minutes.
3. The supernatant was discarded, 1 mL of Advanced DMEM/F-12 (Thermo Fisher Scientific) was added, and the cells were suspended by pipetting.
4. The cell suspension was transferred to a new 1.5 mL tube and centrifuged at 500 g for 5 minutes.
5. The supernatant was discarded, the volume of the pellet was estimated, and the pellet was tapped on ice for from 2 to 3 minutes.
6. Ice-cooled Matrigel (registered trademark) (Coming) having a volume about 20 times the pellet volume was added, and the solution was gently pipetted several times before returning onto ice.
7. To a non-treated 48-well plate pre-warmed to 37°C, 20 µL of the cell suspension was added dropwise.
8. The suspension was incubated at 37°C for 15 minutes or more.
9. ECSY medium (250 µL) was added, followed by maintenance.

### Preparation of Endometrial Model

An endometrial model was prepared by the following procedure.
1. The medium was removed from a maintenance culture solution of endometrial organoids.
2. DMEMIF-12 (cold) (0.5 mL) was added, and the mixture was pipetted 20 times to suspend the cells.
3. Centrifugation was performed at 500 g for 3 minutes and the supernatant was removed.
4. Matrigel (registered trademark) (Corning) and type I collagen (Cell matrix (registered trademark) Type I-A; Nitta Gelatin) were added (Matrigel/type I collagen = 20/80 (volumetric ratio)). Matrigel alone was added to a control.
5. A cell-containing gel (20 µL) was added dropwise to a non-treated 48-well plate, and the mixture was incubated at 37°C for 30 minutes.
6. ESCY medium was added, and the cells were culture at 37°C with 5% CO₂ concentration. During the culture, the medium was changed at a frequency of from 2 to 3 times per week.
7. On the seventh day of the culture, the medium was replaced with ECSY + EPC medium.

### Example 1

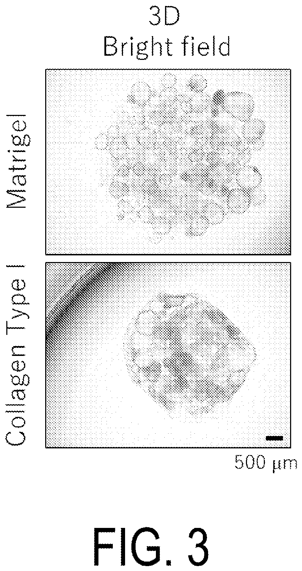

Endometrial models were generated using Matrigel or type I collagen gels by the method described above. FIG. 3 shows bright field phase contrast microscopic images of the endometrial models.

When cultured with Matrigel, the endometrial epithelial cells formed spherical structures inside the Matrigel. The endometrial epithelial cells remained inside the Matrigel and were not exposed on the gel surface.

When cultured using the type 1 collagen gel, the endometrial epithelial cells moved to the gel surface without remaining inside the type I collagen gel. The endometrial epithelial cells were exposed on the gel surface, and contraction of the gel was observed.

### Example 2

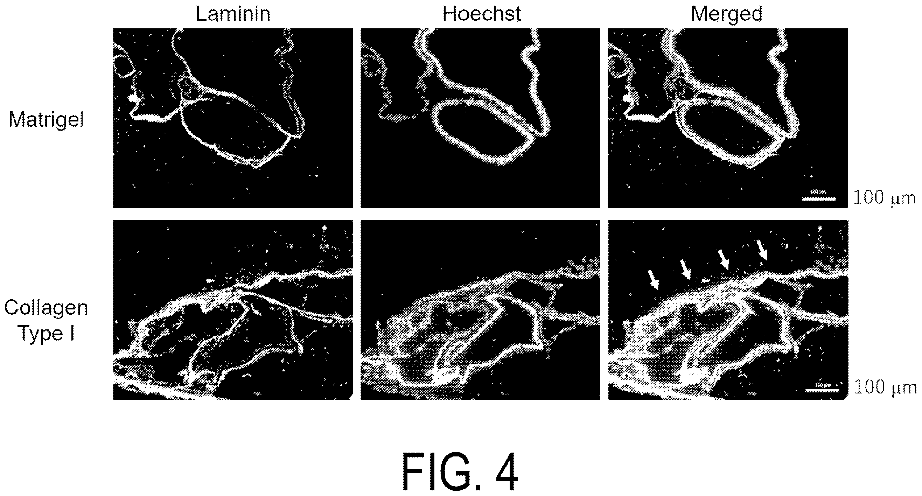

Endometrial models were generated using Matrigel or type I collagen gels by the method described above. Then, sections of the endometrial models were prepared and subjected to immunostaining with an anti-laminin antibody and Hoechst staining. FIG. 4 shows immunostaining images of the endometrial model sections.

When cultured using the Matrigel, the endometrial epithelial cells were wrapped in laminin and were not exposed on the gel surface.

It was confirmed that, when cultured using the type I collagen gel, the endometrial epithelial cells were exposed on the gel surface (arrows).

### Example 3

An endometrial model was prepared using the type I collagen gel in the same manner as described above except that the ECSY + EPC + PLG medium was used instead of the ECSY + EPC medium. Then, a section of the endometrial model was prepared and subjected to immunostaining with an anti-EpCAM antibody or anti-PGR antibody, F-actin staining with phalloidin, and Hoechst staining. EpCAM is an epithelial cell marker and is widely expressed on epithelial cells. F-actin is a structural protein of the cytoskeleton. PGR is a progesterone receptor and is indicative of hormone sensitivity.

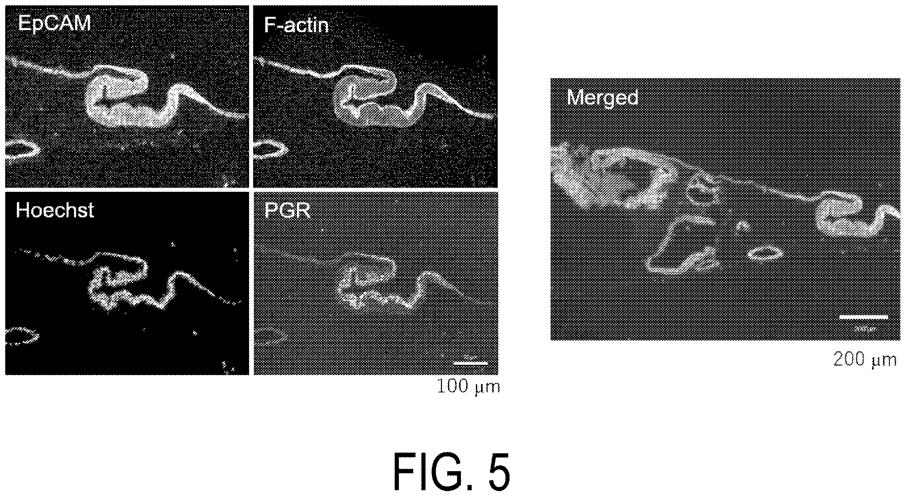

FIG. 5 shows immunostaining images of the endometrial model section. In the endometrial model, a luminal structure and a non-luminal structure were continuously formed. It was confirmed that PGR was expressed in the luminal structure and was hormone-sensitive. The luminal structure had a structure similar to the glandular epithelium of the endometrium.

### Example 4

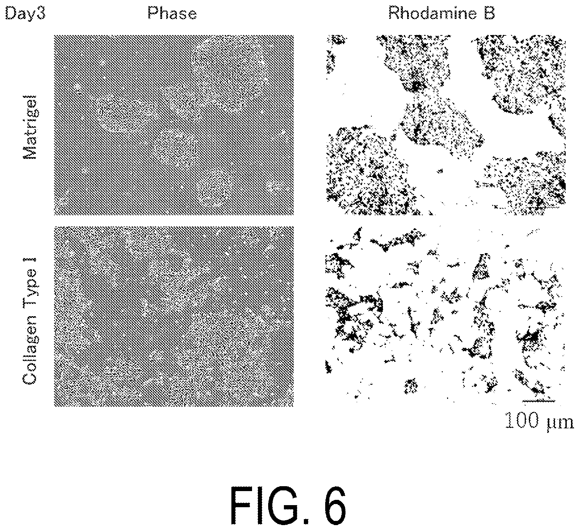

Endometrial cells were plate-cultured using type I collagen gel or Matrigel. FIG. 6 shows phase contrast microscopic images (Phase) of endometrial cells on the third day of the plate culture and images stained with Rhodamine B (Rhocamine B).

When plate-cultured using the Matrigel, the endometrial cells grew in colonies. On the other hand, it was confirmed that, when plate-cultured using the type 1 collagen gel, the endometrial cells were dispersed and migrated radially. This result showed that the migration of the endometrial cells was promoted on the surface of the type 1 collagen gel.

### Example 5

In order to simulate implantation of embryonic cells into the endometrial model, a co-culture test of ES cells (assigned from the National Center for Child Health and Development, UMEZAWA Laboratory) or TS cells with the endometrial model was performed. The TS cells used were TS cells genetically modified to express EGFP (EGFP-TS cells).

The ES cells were induced into blastocyst-like structures and co-cultured with the endometrial models (Yu L et al Nature 2021). The induction into blastocyst-like structures was performed as follows. Naive ES cells were seeded in 256-wells made of agarose at from 25 to 50 cells/well. After overnight culture in a 5i/L/A medium, the medium was replaced with a HDM medium. Three days after the start of the culture in the HDM medium, the medium was replaced with the TDM medium. Thereafter, the culture was continued in the HDM medium while the medium was changed at a frequency of once every two days. From about 10 to about 12 days after seeding of the ES cells, cells that formed a blastocyst-like structure were collected using a mouse pipette and subjected to co-culture with the endometrial model.

The TS cells were subjected to suspension culture in a TS medium (Okae et al Cell Stem Cell 2018) using Petri dishes for 3 days to form aggregates. The aggregates were subjected to co-culture with the endometrial model.

An endometrial model was prepared using the type 1 collagen gel by the method described above. Then, ES cells or EGFP-TS cells were co-cultured with the endometrial model to prepare an endometrial implantation model. An 1VC1 medium and An IVC2 medium were used as the co-culture media. From the start to the first day of the co-culture, the co-culture was performed using the IVC1 medium. On the second day from the start of the co-culture, the medium was replaced with the 1VC2 medium, and co-culture was performed in the IVC2 medium. On the fifth day from the start of the co-culture, sections of the endometrial implantation model were prepared. The sections were subjected to immunostaining with an anti-OCT4 antibody or anti-SDC1 antibody, F-actin staining with phalloidin, and Hoechst staining. OCT4 is an undifferentiation marker and is expressed in undifferentiated cells. SDC1 is an ST cell marker.

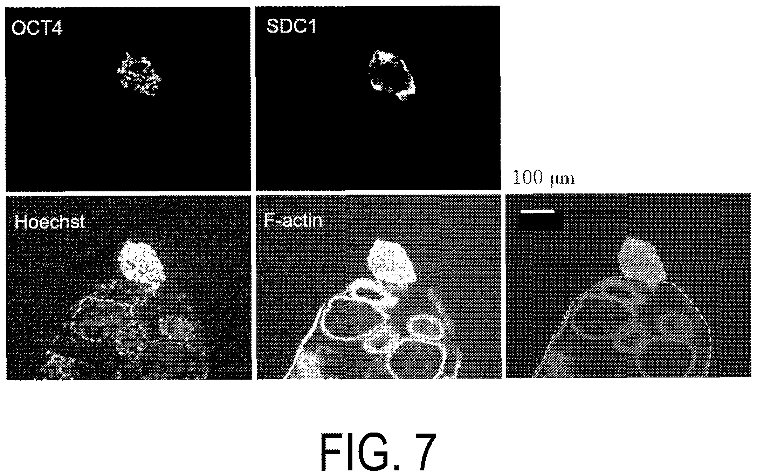

FIG. 7 shows immunostaining images of the sections of the implantation model using the ES cells. FIG. 8 shows immunostaining images of the sections of the implantation model using the EGFP-TS cells. From FIGS. 7 and 8, it was confirmed that the ES cells or EGFP-TS cells adhered to the surface of the endometrial model. In both the ES cells and the TS cells, the expression of SDC1 was confirmed in the cells at the implantation site in contact with the endometrial model. From this result, it was considered that the cells at the implantation site were differentiated into ST cells. From these results, it was shown that implantation of embryonic cells can be simulated using the endometrial models prepared by the above method.

### Industrial Applicability

The present invention provides an endometrial model in which apical surfaces of endometrial epithelial cells are exposed, a method for preparing the endometrial model, and an endometrial implantation model using the endometrial model.

While the preferred embodiments of the invention have been described and illustrated, it is to be understood that they are illustrative of the invention and are not to be considered as limiting. Additions, omissions, substitutions and other changes may be made without departing from the spirit or scope of the invention. Accordingly, the invention is not to be seen as limited by the foregoing description, but is limited only by the scope of the appended claims.

### Reference Signs List

1: Endometrial model
2: Endometrial implantation model
10a, 10b: Endometrial epithelial cell assembly
11: Luminal structure
12: Non-luminal structure
20: Stromal extracellular matrix-containing gel
30: Embryonic cell

## Claims

1. An endometrial model comprising:
a stromal extracellular matrix-containing gel; and
endometrial epithelial cells,
wherein at least some of the endometrial epithelial cells are exposed on a surface of the gel, and
the at least some of the endometrial epithelial cells exposed on the surface of the gel continuously form a luminal structure and a non-luminal structure.

2. The endometrial model according to claim 1, wherein the non-luminal structure is a flat membranous structure.

3. The endometrial model according to claim 1 or 2, wherein the stromal extracellular matrix is type 1 collagen.

4. The endometrial model according to any one of claims 1 to 3, wherein some of the endometrial epithelial cells are present inside the gel.

5. A method for preparing an endometrial model, the method comprising:
(a) allowing endometrial epithelial cells to be present inside a stromal extracellular matrix-containing gel; and
(b) after the step (a), culturing the endometrial epithelial cells.

6. The method for preparing an endometrial model according to claim 4, wherein the stromal extracellular matrix is type I collagen.

7. An endometrial implantation model comprising:
the endometrial model described in any one of claims 1 to 4; and
an embryonic cell.

8. The endometrial implantation model according to claim 7, wherein the embryonic cells comprise at least one type of cells selected from the group consisting of trophoblast stem cells, cytotrophoblast cells, extravillous trophoblast cells, syncytiotrophoblast cells, pluripotent stem cells, and cells derived therefrom.

9. The endometrial implantation model according to claim 7 or 8, wherein the embryonic cells form spheroids or organoids.
